# EUROPEAN PATENT SPECIFICATION

(11) **EP 1 511 489 B1**
(45) Date of publication and mention of the grant of the patent: **02.03.2011**
(21) Application number: 03730069.6
(22) Date of filing: 19.05.2003
(51) Int. Cl.: A61K 31/496, A61K 9/20, A61P 25/16, A61P 25/28, A61P 15/00

(54) **NEW PHARMACEUTICAL COMPOSITIONS CONTAINING FLIBANSERIN POLYMORPH A**
NEUE PHARMAZEUTISCHE ZUSAMMENSTEZUNGEN, FLIBANSERIN POLYMORPH A ENTHALTEND
NOUVELLES COMPOSITIONS PHARMACEUTIQUES CONTENANT UN POLYMORPHE A DE FLIBANSERINE

(30) Priority: 22.05.2002 EP 02011224
(43) Date of publication of application: 09.03.2005
(73) Proprietor: Boehringer Ingelheim Pharma GmbH & Co.KG, 55216 Ingelheim am Rhein (DE)
(72) Inventor: FRIEDL, Thomas, 88416 OCHSENHAUSEN (DE); RADTKE, Guido, Bernhard, Edmund, 55435 Gau-Algesheim (DE)
(86) International application number: PCT/EP2003/005226
(87) International publication number: WO 2003/097058

(56) References cited:
- EP-A- 0 526 434
- WO-A-03/013539
- WO-A-03/014079
- GIRON ET AL: "Thermal analysis and calorimetric methods in the characterisation of polymorphs and solvates" THERMOCHIMICA ACTA, ELSEVIER SCIENCE PUBLISHERS, AMSTERDAM, NL, vol. 248, 1995, pages 1-59, XP002101808 ISSN: 0040-6031

## Description

The invention relates to oral pharmaceutical compositions containing flibanserin, methods for the preparation thereof and use thereof as a medicament.

### Background of the invention

The compound 1-[2-(4-(3-trifluoromethyl-phenyl)piperazin-1-yl)ethyl]-2,3-dihydro-1H-benzimidazol-2-one (flibanserin) is disclosed in form of its hydrochlorid in European Patent Application EP-A-526434 and has the following chemical structure:

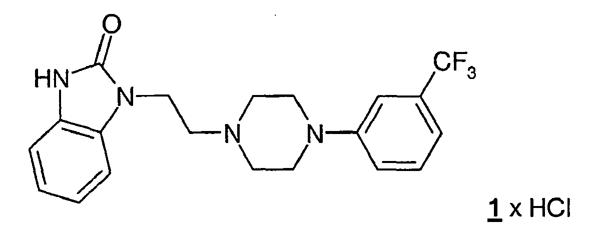

Flibanserin shows affinity for the 5-HT_{1A} and 5-HT₂-receptor. It is therefore a promising therapeutic agent for the treatment of a variety of diseases, for instance depression, schizophrenia, Parkinson, anxiety, sleep disturbances, sexual and mental disorders and age associated memory impairment.

PCT applications WO 03/014079 and WO 03/013539 are state of the art according to Article 54(3) EPC. WO 03/014079 describes pharmaceutical compositions comprising flibanserin polymorph A. WO 03/013539 discloses tablets containing flibanserin.

A certain pharmaceutical activity is of course the basic prerequisite to be fulfilled by a pharmaceutically active agent before same is approved as a medicament on the market. However, there are a variety of additional requirements a pharmaceutically active agent has to comply with. These requirements are based on various parameters which are connected with the nature of the active substance itself. Without being restrictive, examples of these parameters are the stability of the active agent under various environmental conditions, its stability during production of the pharmaceutical formulation and the stability of the active agent in the final medicament compositions. The pharmaceutically active substance used for preparing the pharmaceutical compositions should be as pure as possible and its stability in long-term storage must be guaranteed under various environmental conditions. This is absolutely essential to prevent the use of pharmaceutical compositions which contain, in addition to the actual active substance, degradation products thereof, for example. In such cases the content of active substance in the medicament might be less than that specified.

Uniform distribution of the active substance in the formulation is a critical factor, particularly when the medicament has to be given in low doses. To ensure uniform distribution, the particle size of the active substance can be reduced to a suitable level, e.g. by grinding. Since degradation and/or amorphization of the pharmaceutically active substance as a side effect of the grinding (or micronising) has to be avoided as far as possible, in spite of the hard conditions required during the process, it is absolutely essential that the active substance should be highly stable throughout the grinding process, Only if the active substance is sufficiently stable during the grinding process is it possible to produce a homogeneous pharmaceutical formulation which always contains the specified amount of active substance in reproducible manner.

Finally, the properties of the pharmaceutical composition as such decisively contribute to the bioavailability of the active agent and hence efficacy of the medicament in the intended medical use.

The aim of the invention is thus to provide a new formulation for oral administration containing flibanserin which meets the stringent requirements imposed on pharmaceutical compositions as mentioned above.

### Description of the Invention

It has been found, surprisingly, that the free base of flibanserin in a specific polymophic form best fulfils the requirements to be met within the formulation according to the invention. This specific polymorphic form (polymorph A) is obtainable by specific reaction conditions which are described in more detail hereinbelow. Among other features this polymorphic form is characterized by an endothermic maximum at 161°C which occurs during thermal analysis using DSC (Differential Scanning Calorimetry).

The pharmaceutical composition according to the invention is a tablet for oral administration comprising a core, containing flibanserin polymorph A being characterized by an endothermic maximum at 161°C determined by DSC in admixture with at least one pharmaceutically acceptable excipient and further comprising a film coating comprising titon dioxide and/or talc enveloping said core.

Based on the total mass of the core of the film-coated tablets according to the invention flibanserin polymorph A is present in amounts of 1 to 50 wt.%, preferably 5 to 45 wt.%, particularly preferably about 10 to 40 wt.%. Particularly preferably, the proportion of flibanserin polymorph A is between 15 and 35 wt.%, more preferably between 17 and 32 wt.% based on the total mass of the core.

The core of the pharmaceutical formulation according to the invention contains, in addition to flibanserin polymorph A, at least one excipient as filler/dry binder. Within the scope of the present invention typical fillers are for example lactose monohydrate, both fine milled material or modified lactose like spray-dried lactose and agglomerated lactose (Tablettose), anhydrous lactose,microcrystalline cellulose, dibasic calcium phosphate, cornstarch, sugar alcohols like e.g. mannitol and sorbitol and mixtures thereof. Preferably the filler within the formulation according to the invention is selected from the group consisting of lactose types, microcrystalline cellulose, cornstarch, sugar alcohols and mixtures thereof. More preferably the filler in the formulation according to the invention is selected from the group consisting of lactose types, microcrystalline cellulose, and mixtures thereof. If lactose is used as a filler it is preferably applied in form of the lactose monohydrate fine milled material (e.g. 200 mesh grade).

The core of the film-coated tablet according to the invention may also contain dry and/or wet binding agents, such as povidone (e.g. Kollidon K 25), copovidone (e.g. Kollidon VA 64), hydroxypropyl methylcellulose, hydroxypropylcellulose, corn starch and mixtures thereof. Preferably the binding agent is selected from the group of povidone, hydroxypropyl methylcellulose, hydroxypropyl ethylcellulose, hydroxypropylcellulose, and mixtures thereof. Most preferably hydroxyproypl methylcellulose is selected as binding agent. If hydroxypropyl methylcellulose (HPMC) is applied the HPMC polymers HPMC USP2910 and USP2208 like for instance Methocel E5, E4M, E15M, (K15M, and K100M) supplied for instance by the Dow Chemical Company are of special interest. In the aforementioned abbreviations the designation "E" refers to USP291 0 whereas "K" refers to USP2208. The number designation refers to the viscosity in a 2% aqueous solution (e.g. 5 designates a viscosity of 5 cps; 15M designates a viscosity of 15000 cps).

Based on the total mass of the core of the film-coated tablets according to the invention the filler is preferably present in amounts of 50 to 99 wt.%, preferably 55 to 95 wt.%, particularly preferably about 60 to 90 wt.%. Particularly preferably, the proportion of the total amount of filler is between 65 and 85 wt.%, more preferably between 68 and 80 wt.% based on the total mass of the core.

Preferably the core of the tablet formulation according to the invention comprises flibanserin polymorph A in admixture with lactose monohydrate as the pharmaceutically acceptable excipient.

More preferably the core of the tablet formulation according to the invention comprises flibanserin polymorph A in admixture with lactose monohydrate and microcrystalline cellulose as pharmaceutically acceptable excipients. In formulations according to the invention containing a mixture of lactose monohydrate and microcrystalline cellulose as filler components (or pharmaceutically acceptable excipients), the ratio of lactose monohydrate to microcrystalline cellulose is for example in the range of about 15:1 to about 1:5, preferably in a range of about 10:1 to about 1:3, more preferably in a range of about 6:1 to about 1:1.

In another preferred embodiment according to the invention tablet formulation comprises flibanserin polymorph A in admixture with lactose monohydrate, microcrystalline cellulose and HPMC as pharmaceutically acceptable excipients. In particular preferred formulations according to the invention containing a mixture of lactose monohydrate, microcrystalline cellulose and HPMC as filler/binder components (or pharmaceutically acceptable excipients), the amount of lactose monohydrate is for example in the range of 50 to 95 wt.%, preferably 60 to 90 wt.%, more preferably about 65 to 85 wt.% based on the total mass of the filler/binder used for the preparation of the core. In a particularly preferred embodiment these tablet formulations contain lactose monohydrate in an amount of about 70 to 80 wt.% based on the total mass of the filler/binder used for the preparation of the core. In the particular preferred formulations according to the invention containing a mixture of lactose monohydrate, microcrystalline cellulose and HPMC as filler/binder components (or pharmaceutically acceptable excipients), the amount of microcrystalline cellulose is for example in the range of 5 to 45 wt.%, preferably 15 to 35 wt.%, more preferably about 20 to 30 wt.% based on the total mass of the filler/binder used for the preparation of the core. In a particularly preferred embodiment these tablet formulations contain microcrystalline cellulose in an amount of about 22 to 28 wt.% based on the total mass of the filler/binder used for the preparation of the core. In the particularly preferred formulations according to the invention containing a mixture of lactose monohydrate, microcrystalline cellulose and HPMC as filler/binder components (or pharmaceutically acceptable excipients), the amount of HPMC is for example in the range of 0.5 to 5 wt.%, preferably 1.0 to 4.5 wt.% based on the total mass of the filler/binder used for the preparation of the core. In a particularly preferred embodiment these tablet formulations contain HPMC in an amount of about 1 to 3 wt.% based on the total mass of the filler/binder used for the preparation of the core.

The core of the film-coated tablet according to the invention may also contain disintegrants in addition to the ingredients mentioned above. Within the scope of the present invention these disintegrants may optionally also be known as breakdown agents. These are preferably selected according to the invention from among sodium starch glycolate, crospovidone, croscarmellose sodium, sodium-carboxymethylcellulose, dried corn starch and mixtures thereof. Particularly preferably, within the scope of the present invention, sodium starch glycolate, crospovidone, sodium-carboxymethylcellulose and croscarmellose sodium, preferably croscarmellose sodium are used. If the abovementioned disintegrants are used, the amount by weight used based on the total mass of the core of the film-coated tablet according to the invention is for example in a range from about 0.1 - 10 wt.%, preferably about 0.5 - 5 wt.%, more preferably about 1 - 3 wt.%.

The core of the film-coated tablet according to the invention may also contain flow regulators as additional ingredients. Flow regulators within the scope of the present invention include, for example, silicon dioxide, talc, magnesium stearate and mixtures thereof. According to the invention silicon dioxide is preferably used, particularly preferably in colloidal, highly dispersed form. If the abovementioned flow regulators are used, the amount by weight thereof based on the total mass of the core of the film-coated tablet according to the invention is preferably in a range from about 0.1 - 5 wt.%, preferably about 0.3 - 2 wt.%, particularly preferably between 0.4 and 1.5 wt.%.

The core of the film-coated tablet according to the invention may also contain flow agents, lubricants and mould release or antiadhesive agents as further ingredients. These include, for example, within the scope of the present invention, stearic acid, magnesium stearate, calcium stearate, sodium stearyl fumarate, glycerol tribehenate, talc and mixtures thereof. According to the invention, stearic acid and magnesium stearate are preferably used. If one or several of the aforementioned ingredients is used the amount by weight thereof is preferably in a range from about 0.01 - 5 wt.%, preferably about 0.05 - 3 wt.%, particularly preferably about 0.1 - 2 wt.% based on the total mass of the core of the film-coated tablet. Preferably, especially in case of magnesium stearate the amount thereof is in the range of about 0.2 - 1.5 wt.% based on the total mass of the core of the film-coated tablet.

The film coating enveloping the core of the film-coated tablets according to the invention contains at least one or more film-forming agents selected from among hydroxypropylmethylcellulose, hydroxypropylcellulose, methylcellulose, hydroxymethylcellulose, hydroxyethylcellulose and poly(ethylacrylate) methylmethacrylate, the latter in the form of Eudragit NE 30 D, for example. Alternatively, Eudragit RL 30 D or Eudragit E 12.5 may be used, for example. The above ingredients may optionally also be used in the form of mixtures thereof. Preferred film-forming agents are hydroxypropylmethylcellulose, hydroxypropylcellulose, hydroxymethylcellulose and hydroxyethylcellulose, of which hydroxypropylmethylcellulose and hydroxypropylcellulose are particularly preferred as film-forming agents according to the invention. The abovementioned film-forming agents may be used on their own or in the form of the mixtures thereof. If only one of the abovementioned film-forming agents is used, hydroxypropylmethylcellulose is of particular importance in this context within the scope of the present invention. The amount by weight of film-forming agents based on the total mass of the film coating of the film-coated tablet according to the invention is preferably in a range from about 20 - 95 wt.%, preferably 30 - 90 wt.%.

The film coating enveloping the core may contain emulsifiers and/or plasticisers such as, for example, polyethyleneglycol, glycerol and propyleneglycol, optionally in the form of the mixtures thereof. Preferably, polyethyleneglycols are used as plasticisers. Without restricting the subject matter of the invention thereto, polyethyleneglycol 400 and polyethyleneglycol 6000 are examples of particularly preferred polyethyleneglycols. Within the description of the instant invention references to the term Macrogol are to be understood as references to the term polyethyleneglycol. The values 400 and 6000 mentioned hereinbefore indicate the average molecular weight of the polyethyleneglycol applied. The amount of plasticiser by weight based on the total mass of the film coating of the film-coated tablet according to the invention is preferably in a range from about 1 - 50 wt.%, preferably 5 - 40 wt.%, particularly preferably 10 - 30 wt.%. Preferably the amount of plasticiser is in a range of about 10 - 25 wt.%, more preferably in a range of about 12 - 18 wt.% based on the total mass of the film coating of the film-coated tablet.

The film coating of the film-coated tablet according to the invention may also contain coloured pigments and pigmenting excipients. Iron oxide, titanium dioxide, talc and mixtures thereof may be mentioned by way of example. In case talc is used the amount thereof is for example in a range from about 5 - 50 wt.%, preferably 10 - 40 wt.%, particularly preferably 15 - 30 wt.% based on the total mass of the film coating of the film-coated tablet. Preferably the amount of talc is in a range of about 15 - 20 wt.% based on the total mass of the film coating of the film-coated tablet. In case titanium dioxide is used the amount thereof is for example in a range from about 5 - 55 wt.%, preferably 10 - 40 wt.%, particularly preferably 15 - 35 wt.% based on the total mass of the film coating of the film-coated tablet. Preferably the amount of titanium dioxide is in a range of about 20 - 30 wt.% based on the total mass of the film coating of the film-coated tablet. In case iron oxide is used the amount thereof is for example in a range from about 0.1 - 5 wt.%, preferably about 0.25 - 3 wt.%, more preferably about 0.5 - 1.5 wt.% based on the total mass of the film coating of the film-coated tablet.

In a particular preferred embodiment the film coat enveloping the core of the tablet according to the invention comprises hydroxypropyl methylcellulose, polyethyleneglycol and titanium dioxide. In another embodiment according to the invention the film coat enveloping the core of the tablet according to the invention comprises hydroxypropyl methylcellulose, polyethyleneglycol, titanium dioxide and talc. In yet another embodiment according to the invention the film coat enveloping the core of the tablet according to the invention comprises hydroxypropyl methylcellulose, polyethyleneglycol, titanium dioxide, talc and iron oxides, preferably iron oxide red.

The pharmaceutical composition according to the invention can be prepared according to the procedure outlined in detail in the experimental section of this patent application.

In the light of the pharmaceutical efficacy of flibanserin, the present invention furthermore relates to the use of the flibanserin polymorph A containing formulations according to the invention as a medicament.

A further aspect of the present invention relates to the use of the flibanserin polymorph A containing formulations according to the invention for treating a disease selected from depression, schizophrenia, Parkinson, anxiety, sleep disturbances, sexual and mental disorders and age associated memory impairment.

In particular, the instant invention relates to the use of the flibanserin polymorph A containing formulations according to the invention for the treatment of disorders of sexual desire.

In a preferred embodiment the invention relates to the use of the flibanserin polymorph A containing formulations according to the invention for the treatment of disorders selected from the group consisting of Hypoactive Sexual Desire Disorder, loss of sexual desire, lack of sexual desire, decreased sexual desire, inhibited sexual desire, loss of libido, libido disturbance, and frigidity.

Particular preferred according to the invention are flibanserin polymorph A containing formulations according to the invention for use in the treatment of disorders selected from the group consiting of Hypoactive Sexual Desire Disorder, loss of sexual desire, lack of sexual desire, decreased sexual desire, inhibited sexual desire. In a particularily preferred embodiment the invention relates to the use of the flibanserin polymorph A containing formulations according to the invention for the treatment of disorders selected from the group of Hypoactive Sexual Desire Disorder and loss of sexual desire.

A further aspect of the present invention relates to a method for treating a disease selected from depression, schizophrenia, Parkinson, anxiety, sleep disturbances, sexual and mental disorders and age associated memory impairment comprising the administration of a flibanserin polymorph A containing formulations according to the invention.

In particular, the instant invention relates to a method for the treatment of disorders of sexual desire comprising the administration of a flibanserin polymorph A containing formulations according to the invention.

In a preferred embodiment the invention relates to a method for the treatment of disorders selected from the group consisting of Hypoactive Sexual Desire Disorder, loss of sexual desire, lack of sexual desire, decreased sexual desire, inhibited sexual desire, loss of libido, libido disturbance, and frigidity, comprising the administration of a flibanserin polymorph A containing formulations according to the invention.

Particular preferred according to the invention is a flibanserin polymorph A containing formulations according to the invention for use in the treatment of disorders selected from the group consiting of Hypoactive Sexual Desire Disorder, loss of sexual desire, lack of sexual desire, decreased sexual desire, inhibited sexual desire.

In a particularily preferred embodiment the invention relates to a method for the treatment of disorders selected from the group of Hypoactive Sexual Desire Disorder and loss of sexual desire, comprising the administration of a flibanserin polymorph A containing formulations according to the invention.

The aforementioned therapeutic effects of the flibanserin polymorph A containing formulations according to the invention can be achieved in men and women. However, according to a further aspect of the invention the flibanserin polymorph A containing formulations according to the invention for use in the treatment of female sexual dysfunction are preferred.

The beneficial effects of the flibanserin polymorph A containing formulations according to the invention can be observed regardless of whether the disturbance existed lifelong or was acquired, and independent of etiologic origin (organic - both, physically and drug induced-, psychogen, a combination of organic - both, physically and drug induced-, and psychogen, or unknown).

The invention will be further described by the following examples. These examples disclose certain preferred embodiments of the invention. Accordingly, it is intended that the invention be not limited to the following explicitly disclosed examples.

### Synthesis of flibanserin polymorph A:

375 kg of 1-[(3-trifluoromethyl)phenyl]-4-(2-cloroethyl)piperazin are charged in a reactor with 2500 kg of water and 200 kg of aqueous Sodium Hydroxide 45%. Under stirring 169.2 kg of 1-(2-propenyl)-1,3-dihydro-benzimidazol-2H-one, 780 kg of isopropanol, 2000 kg of water and 220 kg of aqueous Sodium Hydroxide 45% are added. The reaction mixture is heated to 75-85°C and 160 kg of concentrated hydrochloric acid and 200 kg of water are added. The reaction mixture is stirred at constant temperature for about 45 minutes. After distillation of a mixture of water and Isopropanol (about 3000 kg) the remaining residue is cooled to about 65-75°C and the pH is adjusted to 6.5 - 7.5 by addition of 125 kg of aqueous Sodium Hydroxide 45%. After cooling to a temperature of 45-50°C, the pH value is adjusted to 8-9 by addition of about 4 kg of aqueous Sodium Hydroxide 45%.Subsequently the mixture is cooled to 30-35°C and centrifuged. The residue thus obtained is washed with 340 l of water and 126 l of isopropanol and then with water until chlorides elimination. The wet product is dried under vacuum at a temperature of about 45-55°C which leads to 358 kg of crude flibanserin polymorph A. The crude product thus obtained is loaded in a reactor with 1750 kg of Acetone and the resulting mixture is heated under stirring until reflux. The obtained solution is filtered and the filtrate is concentrated by distillation. The temperature is maintained for about 1 hour 0-5°C, then the precipitate solid is isolated by filtration and dried at 55°C for at least 12 hours. The final yield is 280 kg of pure flibanserin polymorph A.

### Characterisation of flibanserin polymorph A:

Flibanserin polymorph A was characterised by DSC (Differantial Scanning Calorimetry). The peak temperature determined for polymorph A is about 161°C. For the characterization via DSC a Mettler TA 3000 System equipped with TC 10-A processor and DSC 20 cell was applied. The heating rate was 10 K/min.

The flibanserin polymorph A was additionally characterised by powder x-ray diffractometry. The x-ray powder diffraction pattern for polymorph A was obtained according to the following conditions:

| | | |
|---|---|---|
| Equipment: | Philips PW 1800/10 diffractometer equipped with a digital microvax 2000. | |
| | | |
| Setting parameters: | X-ray | |
| | Type tube: | Cu (long fine focus) |
| | Wavelenghts (X): | K_{α1} = 1.54060 Å |
| | | K_{α2} = 1.54439 Å |
| | Intensity ratio (α2/α1): | 0.500 |
| | | |
| | Start angle [°2Θ]: | 2.000 |
| | End angle [°2Θ]: | 60.000 |
| | Step size [°2Θ]: | 0.020 |
| | Maximum intensity[s]: | 7310.250 |
| | | |
| | Type of scan: | continuous |
| | | |
| | Minimum peak tip width: | 0.00 |
| | Maximum peak tip width: | 1.00 |
| | Peak base width: | 2.00 |
| | Minimum significance: | 0.75 |
| | Number of peaks: | 69 |
| | | |
| Generator: | high voltage: | 50 KV |
| | tube current: | 30 mA |

The powder x-ray diffraction pattern obtained for polymorph A is illustrated in figure 1. The appropiate values are collected in table 1.

**Table 1:**

| Angle [°2Θ] | d-value α1 [Å] | d-value α 2 [Å] | Peak width [°2Θ] | Peak int [counts] | Back. int [counts] | Rel. int [%] | Signif. |
|---|---|---|---|---|---|---|---|
| 5.195 | 16.9967 | 17.0390 | 0.960 | 8 | 69 | 0.1 | 1.05 |
| 9.045 | 9.7689 | 9.7931 | 0.100 | 92 | 96 | 1.3 | 0.97 |
| 9.335 | 9.4660 | 9.4896 | 0.080 | 114 | 98 | 1.6 | 0.88 |
| 10.025 | 8.8160 | 8.8379 | 0.140 | 400 | 100 | 5.5 | 7.18 |
| 10.595 | 8.3430 | 8.3637 | 0.140 | 204 | 102 | 2.8 | 3.46 |
| 11.290 | 7.8309 | 7.8503 | 0.140 | 467 | 104 | 6.4 | 6.91 |
| 13.225 | 6.6891 | 6.7058 | 0.180 | 548 | 112 | 7.5 | 13.10 |
| 14.595 | 6.0642 | 6.0793 | 0.180 | 404 | 121 | 5.5 | 9.17 |
| 15.460 | 5.7268 | 5.7410 | 0,140 | 4186 | 125 | 57.3 | 23.20 |
| 16.655 | 5.3185 | 5.3317 | 0.200 | 515 | 130 | 7.0 | 12.38 |
| 17.085 | 5.1856 | 5.1985 | 0.100 | 1347 | 132 | 18.4 | 2.78 |
| 17.285 | 5.1260 | 5.1388 | 0.060 | 1399 | 135 | 19.1 | 2.26 |
| 17.420 | 5.0866 | 5.0992 | 0.100 | 1204 | 135 | 16.5 | 4.71 |
| 18.140 | 4.8863 | 4.8984 | 0.180 | 1043 | 139 | 14.3 | 13.14 |
| 18.650 | 4.7538 | 4.7656 | 0.120 | 1063 | 142 | 14.5 | 0.91 |
| 19.140 | 4.6332 | 4.6447 | 0.140 | 7310 | 144 | 100.0 | 32.77 |
| 19.820 | 4.4757 | 4.4869 | 0.160 | 3624 | 146 | 49.6 | 9.02 |
| 20.080 | 4.4184 | 4.4294 | 0.140 | 5402 | 149 | 73.9 | 21.06 |
| 20.385 | 4.3530 | 4.3638 | 0.160 | 2652 | 149 | 36.3 | 23.25 |
| 21.215 | 4.1845 | 4.1949 | 0.160 | 369 | 154 | 5.0 | 5.78 |
| 21.890 | 4.0570 | 4.0670 | 0.200 | 773 | 156 | 10.6 | 3.09 |
| 22.630 | 3.9259 | 3.9357 | 0.280 | 4277 | 161 | 58.5 | 74.66 |
| 23.210 | 3.8291 | 3.8386 | 0.120 | 484 | 164 | 6.6 | 3.33 |
| 24.355 | 3.6516 | 3.6607 | 0.060 | 2725 | 169 | 37.3 | 1.16 |
| 24.610 | 3.6144 | 3.6234 | 0.140 | 3540 | 172 | 48.4 | 17.08 |
| 24.995 | 3.5596 | 3.5684 | 0.100 | 529 | 174 | 7.2 | 1.01 |
| 25.260 | 3.5228 | 3.5316 | 0.120 | 557 | 174 | 7.6 | 3.02 |
| 26.575 | 3.3514 | 3.3597 | 0.240 | 2421 | 182 | 33.1 | 42.58 |
| 27.155 | 3.2811 | 3.2893 | 0.140 | 676 | 185 | 9.2 | 1.32 |
| 27.310 | 3.2629 | 3.2710 | 0.100 | 767 | 185 | 10.5 | 2.75 |
| 27.865 | 3.1991 | 3.2071 | 0.120 | 420 | 188 | 5.7 | 1.08 |
| 28.210 | 3.1608 | 3.1686 | 0.100 | 1467 | 190 | 20.1 | 0.79 |
| 28.325 | 3.1482 | 3.1560 | 0.140 | 1789 | 190 | 24.5 | 4.41 |
| 28.650 | 3.1132 | 3.1210 | 0.180 | 1204 | 190 | 16.5 | 11.65 |
| 29.520 | 3.0234 | 3.0309 | 0.220 | 1011 | 196 | 13.8 | 15.74 |
| 30.250 | 2.9521 | 2.9594 | 0.120 | 159 | 199 | 2.2 | 1.22 |
| 31.105 | 2.8729 | 2.8800 | 0.360 | 282 | 204 | 3.9 | 8.14 |
| 31.905 | 2.8026 | 2.8096 | 0.100 | 339 | 207 | 4.6 | 0.96 |
| 32.350 | 2.7651 | 2.7720 | 0.120 | 237 | 210 | 3.2 | 3.01 |
| 33.300 | 2.6884 | 2.6950 | 0.180 | 1347 | 216 | 18.4 | 14.06 |
| 33.640 | 2.6620 | 2.6686 | 0.100 | 404 | 216 | 5.5 | 1.45 |
| 34.880 | 2.5701 | 2.5765 | 0.200 | 202 | 222 | 2.8 | 1.04 |
| 35.275 | 2.5422 | 2.5486 | 0.240 | 299 | 225 | 4.1 | 4.84 |
| 36.055 | 2.4890 | 2.4952 | 0.280 | 202 | 228 | 2.8 | 3.78 |
| 36.910 | 2.4333 | 2.4393 | 0.320 | 169 | 234 | 2.3 | 0.90 |
| 37.160 | 2.4175 | 2.4235 | 0.120 | 216 | 234 | 3.0 | 2.14 |
| 37.680 | 2.3853 | 2.3912 | 0.240 | 240 | 237 | 3.3 | 1.58 |
| 39.435 | 2.2831 | 2.2888 | 0.280 | 449 | 246 | 6.1 | 2.67 |
| 39.675 | 2.2698 | 2.2755 | 0.080 | 396 | 246 | 5.4 | 0.82 |
| 40.325 | 2.2347 | 2.2403 | 0.160 | 520 | 250 | 7.1 | 0.95 |
| 40.930 | 2.2031 | 2.2086 | 0.120 | 480 | 253 | 6.6 | 2.66 |
| 41.445 | 2.1769 | 2.1823 | 0.240 | 372 | 256 | 5.1 | 2.65 |
| 41.990 | 2.1499 | 2.1552 | 0.120 | 538 | 259 | 7.4 | 1.31 |
| 42.670 | 2.1172 | 2.1225 | 0.160 | 428 | 262 | 5.9 | 1.45 |
| 43.145 | 2.0950 | 2.1002 | 0.120 | 433 | 266 | 5.9 | 1.50 |
| 44.190 | 2.0478 | 2.0529 | 0.160 | 376 | 269 | 5.1 | 0.89 |
| 46.095 | 1.9675 | 1.9724 | 0.160 | 279 | 279 | 3.8 | 0.86 |
| 46.510 | 1.9509 | 1.9558 | 0.240 | 310 | 282 | 4.2 | 0.87 |
| 48.305 | 1.8826 | 1.8872 | 0.200 | 506 | 292 | 6.9 | 2.06 |
| 48.900 | 1.8610 | 1.8657 | 0.240 | 615 | 296 | 8.4 | 1.67 |
| 50.330 | 1.8115 | 1.8160 | 0.160 | 437 | 303 | 6.0 | 1.73 |
| 51.035 | 1.7881 | 1.7925 | 0.080 | 416 | 306 | 5.7 | 0.93 |
| 53.550 | 1.7099 | 1.7141 | 0.480 | 177 | 317 | 2.4 | 2.84 |
| 54.500 | 1.6823 | 1.6865 | 0.400 | 130 | 324 | 1.8 | 1.37 |
| 55.420 | 1.6565 | 1.6606 | 0.320 | 130 | 328 | 1.8 | 1.72 |
| 56.220 | 1.6348 | 1.6389 | 0.320 | 121 | 331 | 1.7 | 0.87 |
| 56.770 | 1.6203 | 1.6243 | 0.240 | 142 | 335 | 1.9 | 1.59 |
| 57.405 | 1.6039 | 1.6079 | 0.240 | 112 | 339 | 1.5 | 1.19 |
| 58.500 | 1.5764 | 1.5804 | 0.240 | 67 | 342 | 0.9 | 1.57 |

### Manufacturing of flibanserin containing film-coated tablets:

### A) Equipment used:

The following equipment was used in the method of preparation of the pharmaceutical composition according to the invention:
Mixing vessel with Ekato stirrer and Ultra Turrax for granulation liquid and film coating suspension;
high shear mixer/granulator (e.g. Diosna P 400);
wet screen machine (e.g. Alexanderwerk);
fluid bed dryer (e.g. Glatt WSG 15);
dry screen machine (e.g. Quadro Cornil AS 197);
free fall blender (e.g. Servolift 120 l or container mixer);
rotary tablet press (e.g. Fette P 1200);
film coater (e.g. Glatt GC 1250);

### B) Process description:

As a first step the granulation liquid for the wet granulation process ist prepared. Purified water is filled into a suitable mixing vessel and heated to about 80°C. Then Hypromellose (Methocel E5 Prem) and/or additional wet binding components are stirred in, and the dispersion is cooled down to room temperature. If necessary, the liquid is allowed to stand overnight (completeness of solution / reduction of frothing) and stirred up before use. If necessary, any weight loss is compensated with purified water. The dry matter (soldis content) of this granulation liquid is preferrably in the range of 4-6 %.

For the granulation process Lactose monohydrate, fine milled and sieved, the required quantity of Flibanserin polymorph A (depending on the dose strength), micronized quality, and Cellulose, microcrystalline (Avicel PH 101) are filled in in this order, mixed homogeneously for about 4 minutes using impeller and chopper blades. Next the granulation liquid is added either manually or by spray nozzles and the wet mass is granulated for about 2-3 minutes, again using impeller and chopper blades. After discharging of the high shear mixer/granulator the wet granules are wet-screened through a 3.0 mm mesh size sieve to destroy large agglomerates. The wet-screened material is transferred to a conventional fluid bed drier (or alternatively to a tray drier) and dried at an inlet air temperature of approximately 100 °C until an exhaust air temperature (or alternatively product temperature) of approximately 50°C (45-55°C) is reached. The residual moisture of the granulate in terms of loss on drying should be in the range of 0.5-1.5 %. The dried granules are then dry screened with the help of a Cornil screen machine using a 2 mm rasp screen. Finally, the screened granulate is filled into a suitable free-fall blender, e.g. a container mixer, the crosslinked Carboxymethylcellulose sodium (Croscarmellose sodium, brand name: Ac-Di-Sol) and Magnesium stearate are added, and the components are mixed for 10-20 minutes, preferrably 15 minutes, at a mixing speed of 10 rpm until homogeneous.

The final tableting mixture is compressed on a suitable tablets press (e.g. rotary press) to the respective target weight of the required dose strength of Flibanserin tablets using the appropriate tools (e.g. in case of 50 mg tablets: 9 mm round, biconvex, with bevelled edges; or in case of 100 mg tablets: 14x6.8 mm oblong shaped). Predetermined hardness specifications for the different tool dimensions have to be followed in order to achieve the intended drug dissolution profile and product characteristics.

Since the drug substance Flibanserin is of bitter taste and slightly light sensitive, a protecting film coat has to be applied to the tablet cores in order to achieve a stable and consumer friendly product. To this end a coating suspension is prepared by filling purified water into a suitable mixing vessel, and dissolving polyethyleneglycol 6000 and then hydroxypropylmethylcellulose with the help of a high intensity stirrer. In a next step an aqueous slurry of titanium dioxide, talc and iron oxide red (in case of coloured film tablets) is poured and stirred into the film-forming polymer solution. The dry matter of this coating suspension is in the range of 10-15 %, preferrably about 12-13 %.

The above prepared tablet cores are filled into a suitable film coater (e.g. an Accela Cota with a 36" pan, or a Glatt GC 1250 Coater with perforated pan, and top spray system), and preheated up to a temperature of approximately 50 °C. After this product temperature is reached the coating suspension is sprayed onto the cores with the help of one or more spray nozzles at a spray pressure of about 2 bar, a spray rate of about 4 kg/h (in case of Accela Cota), an inlet air temperature of about 60-85°C. It is important to control and maintain the product temperature during spraying at a level of between 48-52° C to achieve a high quality film-coat. After the spraying is finished the film-coated tablets are cooled down to approx. 30°C before the equipment is discharged. The total process time for the film-coating is in the range of 2-3 hours.

After all in-process and quality controls have been performed the bulk film-coated tablets are now ready for primary packaging into the respective marketing presentations (e.g. PVC/PVDC blister packs or HDPE bottles).

The following film-coated tablets were obtained in analogy to the method of preparation described hereinbefore.

### Example N°1 - Composition

| Core | | |
|---|---|---|
| | **Constituents** | **mg/tablet** |
| | Flibanserin polymorph A | 25.000 |
| | Lactose monohydrate | 71.720 |
| | Microcrystalline cellulose | 23.905 |
| | HPMC (Methocel E5) | 1.250 |
| | Carboxymethylcellulose sodium | 2.500 |
| | Magnesium stearate | 0.625 |

| Coating | | |
|---|---|---|
| | **Constituents** | **mg/ tablet** |
| | HPMC (Methocel E5) | 1.440 |
| | Polyethylene Glycol 6000 | 0.420 |
| | Titanium dioxide | 0.600 |
| | Talc | 0.514 |
| | Iron oxide red | 0.026 |
| | | |
| | **Total Film coated tablet** | **128.000** |

### Example N°2 - Composition

| Core | | |
|---|---|---|
| | **Constituents** | **mg/tablet** |
| | Flibanserin polymorph A | 50.000 |
| | Lactose monohydrate | 143.440 |
| | Microcrystalline cellulose | 47.810 |
| | HPMC (e.g. Pharmacoat 606) | 2.500 |
| | Carboxymethylcellulose sodium | 5.000 |
| | Magnesium stearate | 1.250 |

| Coating | | |
|---|---|---|
| | **Constituents** | **mg/ tablet** |
| | HPMC (e.g. Pharmacoat 606) | 2.400 |
| | Polyethylene Glycol 6000 | 0.700 |
| | Titanium dioxide | 1.000 |
| | Talc | 0.857 |
| | Iron oxide red | 0.043 |
| | | |
| | **Total Film coated tablet** | **255.000** |

### Example N°3 - Composition

| Core | | |
|---|---|---|
| | **Constituents** | **mg/tablet** |
| | Flibanserin polymorph A | 100.000 |
| | Lactose monohydrate | 171.080 |
| | Microcrystalline cellulose | 57.020 |
| | HPMC (e.g. Methocel E5) | 3.400 |
| | Carboxymethylcellulose sodium | 6.800 |
| | Magnesium stearate | 1.700 |

| Coating | | |
|---|---|---|
| | **Constituents** | **mg/ tablet** |
| | HPMC (e.g. Methocel E5) | 3.360 |
| | Polyethylene Glycol 6000 | 0.980 |
| | Titanium dioxide | 1.400 |
| | Talc | 1.200 |
| | Iron oxide red | 0.060 |
| | | |
| | Total Film coated tablet | 1347.000 |

### Example N°4 - Composition

| Core | | |
|---|---|---|
| | **Constituents** | **mg/tablet** |
| | Flibanserin polymorph A | 2.000 |
| | Dibasic Calciumphosphate, anhydrous | 61.010 |
| | Microcrystalline cellulose | 61.010 |
| | HPMC (Methocel E5) | 1.950 |
| | Carboxymethylcellulose sodium | 2.600 |
| | Colloidal silicon dioxide | 0.650 |
| | Magnesium stearate | 0.780 |

| Coating | | |
|---|---|---|
| | **Constituents** | **mg/ tablet** |
| | HPMC (Methocel E5) | 1.440 |
| | Polyethylene Glycol 6000 | 0.420 |
| | Titanium dioxide | 0.600 |
| | Talc | 0.514 |
| | Iron oxide red | 0.026 |
| | | |
| | **Total Film coated tablet** | **133.000** |

### Example N°5 - Composition

| Core | | |
|---|---|---|
| | **Constituents** | **mg/tablet** |
| | Flibanserin polymorph A | 100.000 |
| | Dibasic Calciumphosphate, anhydrous | 69.750 |
| | Microcrystalline cellulose | 69.750 |
| | HPMC (e.g. Methocel E5) | 2.750 |
| | Carboxymethylcellulose sodium | 5.000 |
| | Colloidal silicon dioxide | 1.250 |
| | Magnesium stearate | 1.500 |

| Coating | | |
|---|---|---|
| | **Constituents** | **mg/ tablet** |
| | HPMC (e.g. Methocel E5) | 2.400 |
| | Polyethylene Glycol 6000 | 0.700 |
| | Titanium dioxide | 1.043 |
| | Talc | 0.857 |
| | | |
| | **Total Film coated tablet** | **255.000** |

### Example N°6 - Composition

| Core | | |
|---|---|---|
| | **Constituents** | **mg/tablet** |
| | Flibanserin polymorph A | 20.000 |
| | Lactose monohydrate | 130.000 |
| | Microcrystalline cellulose | 43.100 |
| | Hydroxypropyl Cellulose (e.g. Klucel LF) | 1.900 |
| | Sodium Starch Glycolate | 4.000 |
| | Magnesium stearate | 1.000 |

| Coating | | |
|---|---|---|
| | **Constituents** | **mg/ tablet** |
| | HPMC (e.g. Methocel E5) | 2.400 |
| | Polyethylene Glycol 6000 | 0.700 |
| | Titanium dioxide | 1.043 |
| | Talc | 0.857 |
| | | |
| | **Total Film coated tablet** | **205.000** |

## Claims

1. Pharmaceutical composition for oral administration comprising a tablet core, containing flibanserin polymorph A being **characterized by** an endothermic maximum at 161°C determined by DSC in admixture with at least one pharmaceutically acceptable excipient and further comprising a film coating comprising titan dioxide and/or talc, enveloping said tablet core.

2. Pharmaceutical composition according to claim 1, **characterized in that** titan dioxide is present in amounts of 5-55 wt.% based on the total mass of the film coating of the film coated tablet.

3. Pharmaceutical composition according to claim 1 or 2, **characterized in that** talc is present in amounts of 5-50 wt.% based on the total mass of the film coating of the film coated tablet.

4. Pharmaceutical composition according to claim 1 to 3, **characterized in that** the pharmaceutically acceptable excipient is a filler selected from the group consisting of lactose monohydrate, both fine milled material or modified lactose like spray-dried lactose and agglomerated lactose (Tablettose), anhydrous lactose, microcrystalline cellulose, dibasic calcium phosphate, cornstarch, sugar alcohols and mixtures thereof.

5. Pharmaceutical composition according to any of the claims 1 to 4, **characterized in that** flibanserin polymorph A is present in amounts of 1 to 50 wt.% based on the total mass of the core.

6. Pharmaceutical composition according to any of the claims 1 to 5, **characterized in that** the core contains filler in amounts of 50 to 99 wt.% based on the total mass of the core.

7. Pharmaceutical composition according to any of the claims 1 to 6, **characterized in that** the core additionally contains a binding agent selected from the group consisting of povidone, copovidone, hydroxypropyl methylcellulose, hydroxypropylcellulose, corn starch and mixtures thereof.

8. Pharmaceutical composition according to any of the claims 1 to 7, **characterized in that** the core additionally contains a disintegrant selected from among sodium starch glycolate, crospovidone, croscarmellose sodium, sodium-carboxymethylcellulose, dried corn starch and mixtures thereof.

9. Pharmaceutical composition according to any of claims 1 to 8, **characterized in that** the core additionally contains flow regulators, lubricants and mould release or antiadhesive agents selected from the group consisting of silicon dioxide, talc, magnesium stearate and mixtures thereof.

10. Pharmaceutical composition according to any of claims 1 to 9, **characterized in that** the film coating enveloping the core contains at least one film-forming agent selected from among hydroxypropylmethylcellulose, hydroxypropylcellulose, methylcellulose, hydroxymethylcellulose, hydroxyethylcellulose and poly(ethylacrylate) methylmethacrylate.

11. Use of a flibanserin polymorph A containing formulation according to one of claims 1 to 10 for the manufacture of a medicament for the treatment of diseases selected from the group consisiting of depression, schizophrenia, Parkinson, anxiety, sleep disturbances, sexual and mental disorders and age associated memory impairment.

12. Use according to claim 11, **characterized in that** the disease is Hypoactive Sexual Desire Disorder.

## Patentansprüche

1. Pharmazeutische Zusammensetzung zur oralen Verabreichung, umfassend einen Tablettenkern, enthaltend Flibanserin-Polymorph A, charakterisiert durch ein endothermes Maximum bei 161°C, bestimmt durch DSC, in einer Mischung mit mindestens einem pharmazeutisch akzeptablen Hilfsstoff, und weiterhin umfassend eine Filmbeschichtung, umfassend Titandioxid und/oder Talk, die den Tablettenkern einhüllt.

2. Pharmazeutische Zusammensetzung nach Anspruch 1, **dadurch gekennzeichnet, dass** das Titandioxid in Mengen von 5 bis 55 Gew.-%, basierend auf der Gesamtmasse der Filmbeschichtung der filmbeschichteten Tablette, vorhanden ist.

3. Pharmazeutische Zusammensetzung nach Anspruch 1 oder 2, **dadurch gekennzeichnet, dass** der Talk in Mengen von 5 bis 50 Gew.-%, basierend auf der Gesamtmasse der Filmbeschichtung der filmbeschichteten Tablette, vorhanden ist.

4. Pharmazeutische Zusammensetzung nach Anspruch 1 bis 3, **dadurch gekennzeichnet, dass** der pharmazeutisch akzeptable Hilfsstoff einen Füllstoff darstellt, ausgewählt aus der Gruppe, bestehend aus Lactosemonohydrat, als feingemahlenes Material oder modifizierte Lactose, wie sprühgetrocknete Lactose und agglomerierte Lactose (Tablettose), wasserfreier Lactose, mikrokristalliner Cellulose, dibasischem Calciumphosphat, Maisstärke, Zuckeralkoholen und Mischungen davon.

5. Pharmazeutische Zusammensetzung nach irgendeinem der Ansprüche 1 bis 4, **dadurch gekennzeichnet, dass** der Flibanserin-Polymorph A in Mengen von 1 bis 50 Gew.-%, basierend auf der Gesamtmasse des Kerns, vorhanden ist.

6. Pharmazeutische Zusammensetzung nach irgendeinem der Ansprüche 1 bis 5, **dadurch gekennzeichnet, dass** der Kern Füllstoff in Mengen von 50 bis 99 Gew.-%, basierend auf der Gesamtmasse des Kerns, enthält.

7. Pharmazeutische Zusammensetzung nach irgendeinem der Ansprüche 1 bis 6, **dadurch gekennzeichnet, dass** der Kern zusätzlich ein Bindemittel enthält, ausgewählt aus der Gruppe, bestehend aus Povidon, Copovidon, Hydroxypropylmethylcellulose, Hydroxypropylcellulose, Maisstärke und Mischungen davon.

8. Pharmazeutische Zusammensetzung nach irgendeinem der Ansprüche 1 bis 7, **dadurch gekennzeichnet, dass** der Kern zusätzlich ein desintegrierendes Mittel bzw. Auflösungshilfsmittel enthält, ausgewählt aus Natriumstärkeglykolat, Crospovidon, Croscarmellosenatrium, Natriumcarboxymethylcellulose, getrockneter Maisstärke und Mischungen davon.

9. Pharmazeutische Zusammensetzung nach irgendeinem der Ansprüche 1 bis 8, **dadurch gekennzeichnet, dass** der Kern zusätzlich Fließregulatoren, Schmiermittel und Entformungshilfsmittel oder Antiadhäsionsmittel enthält, ausgewählt aus der Gruppe, bestehend aus Siliciumdioxid, Talk, Magnesiumstearat und Mischungen davon.

10. Pharmazeutische Zusammensetzung nach irgendeinem der Ansprüche 1 bis 9, **dadurch gekennzeichnet, dass** die Filmbeschichtung, die den Kern umhüllt bzw. einhüllt, mindestens ein Filmbildungsmittel enthält, ausgewählt aus Hydroxypropylmethylcellulose, Hydroxypropylcellulose, Methylcellulose, Hydroxymethylcellulose, Hydroxyethylcellulose und Poly(ethylacrylat)methylmethacrylat.

11. Verwendung einer den Flibanserin-Polymorph A enthaltenden Formulierung nach einem der Ansprüche 1 bis 10 zur Herstellung eines Arzneimittels zur Behandlung von Krankheiten, ausgewählt aus der Gruppe, bestehend aus Depression, Schizophrenie, Parkinson, Angstzuständen, Schlafstörungen, sexuellen und mentalen Störungen und altersbedingter Gedächtnisstörung.

12. Verwendung nach Anspruch 11, **dadurch gekennzeichnet, dass** die Krankheit hypoaktive sexuelle Begehrensstörung (Hypoactive Sexual Desire Disorder) darstellt.

## Revendications

1. Composition pharmaceutique pour une administration orale composant un noyau de comprimé, contenant le polymorphe A de la flibansérine étant **caractérisé par** un maximum endotherme à 161 °C déterminé par DSC en mélange avec au moins un excipient pharmaceutiquement acceptable et comprenant en outre un enrobage sous forme de film comprenant du dioxyde de titane et/ou du talc, enveloppant ledit noyau de comprimé.

2. Composition pharmaceutique selon la revendication 1, **caractérisée en ce que** le dioxyde de titane est présent dans des quantités de 5 à 55 % en poids en se basant sur la masse totale de l'enrobage sous forme de film du comprimé enrobé d'un film.

3. Composition pharmaceutique selon la revendication 1 ou 2, **caractérisée en ce que** le talc est présent dans des quantités de 5 à 50 % en poids en se basant sur la masse totale de l'enrobage sous forme de film du comprimé enrobé d'un film.

4. Composition pharmaceutique selon la revendication 1 à 3, **caractérisée en ce que** l'excipient pharmaceutiquement acceptable est un agent de remplissage choisi dans le groupe comprenant du lactose monohydraté, à la fois du matériau finement broyé ou du lactose modifié comme du lactose séché par pulvérisation et du lactose aggloméré (Tablettose), du lactose anhydre, de la cellulose microcristalline, du phosphate de calcium dibasique, de l'amidon de maïs, des alcools de sucres et des mélanges de ceux-ci.

5. Composition pharmaceutique selon l'une quelconque des revendications 1 à 4, **caractérisée en ce que** le polymorphe A de la flibansérine est présent dans des quantités de 1 à 50 % en poids en se basant sur la masse totale du noyau.

6. Composition pharmaceutique selon l'une quelconque des revendications 1 à 5, **caractérisée en ce que** le noyau contient un agent de remplissage dans des quantités de 50 à 99 % en poids en se basant sur la masse totale du noyau.

7. Composition pharmaceutique selon l'une quelconque des revendications 1 à 6, **caractérisée en ce que** le noyau contient en outre un agent de liaison choisi dans le groupe comprenant de la povidone, de la copovidone, de l'hydroxypropylméthylcellulose, de l'hydroxypropylcellulose, de l'amidon de maïs et des mélanges de ceux-ci.

8. Composition pharmaceutique selon l'une quelconque des revendications 1 à 7, **caractérisée en ce que** le noyau contient en outre un agent de désagrégation choisi parmi du glycolate d'amidon sodique, de la crospovidone, de la croscarmellose sodique, de la carboxyméthyl-cellulose sodique, de l'amidon de maïs séché et des mélanges de ceux-ci.

9. Composition pharmaceutique selon l'une quelconque des revendications 1 à 8, **caractérisée en ce que** le noyau contient en outre des régulateurs de débit, des lubrifiants et des agents de démoulage ou antiadhésifs choisis dans le groupe comprenant du dioxyde de silicium, du talc, du stéarate de magnésium et des mélanges de ceux-ci.

10. Composition pharmaceutique selon l'une quelconque des revendications 1 à 9, **caractérisée en ce que** l'enrobage sous forme de film enveloppant le noyau contient au moins un agent formant un film choisi parmi l'hydroxypropylméthylcellulose, l'hydroxypropylcellulose, la méthylcellulose, l'hydroxyméthylcellulose, l'hydroxyéthylcellulose et le poly (acrylate d'éthyle) méthacrylate de méthyle.

11. Utilisation d'un polymorphe A de la flibansérine contenant une formulation selon l'une quelconque des revendications 1 à 10 pour la fabrication d'un médicament destiné au traitement de maladies choisies dans le groupe comprenant la dépression, la schizophrénie, la maladie de Parkinson, l'anxiété, les troubles du sommeil, les troubles sexuels et mentaux et l'altération de la mémoire associée à l'âge.

12. Utilisation selon la revendication 11, **caractérisée en ce que** la maladie est le trouble du désir sexuel hypoactif.
